# EUROPEAN PATENT APPLICATION

(11) **EP 1 693 107 A1**
(43) Date of publication of application: **23.08.2006**
(21) Application number: 06250531.8
(22) Date of filing: 01.02.2006
(51) Int. Cl.: B01J 37/34, B01J 23/16, C07C 51/215, C07C 51/25

(54) **Method for preparing mixed-metal oxide catalysts and the catalysts produced thereby**

(30) Priority: 11.02.2005 US 652159 P
(71) Applicant: ROHM AND HAAS COMPANY, Philadelphia, Pennsylvania 19106-2399 (US); The University of Connecticut, Storrs, Connecticut 06269-1133 (US)
(72) Inventor: Gaffney, Anne Mae, Pennsylvania 19380 (US); Espinal, Laura, Connecticut 06066 (US); Le, Dominique Hung Nhu, Pennsylvania 19082 (US); Suib, Steven Lawrence, Connecticut 06268 (US)
(74) Representative: Kent, Venetia Katherine

(57) **Abstract**

Processes for preparing mixed metal oxide catalysts suitable for partial oxidation of alkanes, alkenes and mixtures thereof, where in the processes comprise the steps of: providing an aqueous aerosol of one or more metal oxide catalyst precursors; and irradiating the aqueous aerosol of one or more metal oxide catalyst precursors with microwave energy. Optionally, the catalyst may he further modified using one or more chemical treatments, one or more physical treatments and one or more combinations of chemical and physical treatments, to further improve catalyst performance characteristics.

## Description

The present invention relates to methods for producing mixed metal oxide catalysts useful for catalytically converting alkanes, alkenes and mixtures thereof to their corresponding oxygenates, including unsaturated carboxylic acids and esters thereof, by vapor phase oxidation. In particular, the present invention relates to methods for preparing mixed metal oxide catalysts which involve irradiating mixed metal oxide precursors with microwave energy at microwave frequencies

Catalytic partial oxidation of alkanes and alkenes to unsaturated carboxylic acids and their corresponding esters are important commercial processes. However, efforts to improve the selectivity and efficiency of such processes are ongoing, and sometimes focus on optimization of the mixed metal oxide catalysts used in these processes, as well as the methods of making them.

International Publication No. 99/00326 discloses initiating a redox reaction between a plurality of metal salts in aqueous solution which requires at least one strong oxidizing agent and at least one strong reducing agent using microwave energy, wherein the metal oxide product is produced by establishing the redox couple. This publication, however, fails to disclose any methods for controlling the particle size of the material being irradiated with microwave energy. Furthermore, this publication fails to disclose or suggest the use of microwave energy for successful synthesis of mixed metal oxide catalysts which are suitable for partial oxidation of alkanes, alkenes, and mixtures thereof.

The present invention provides a process for preparing one or more mixed metal oxide catalysts suitable for partial oxidation of alkanes, alkenes, and mixtures thereof, comprising the steps of: a) generating one or more aerosols from one or more solutions comprising at least one metal oxide catalyst precursor; and b) irradiating said one or more aqueous aerosols of said one or more mixed metal oxide precursors with microwave energy, having one or more microwave frequencies. The mixed metal oxide precursor aerosols may be generated using an ultrasonic nozzle.

The present invention also provides a catalyst prepared by the aforesaid process.

The catalyst may comprise a compound having the empirical formula:

MoVₐNb_{b}X_{c}Z_{d}Oₙ

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements.

Calcining of the aforesaid compound may be performed after the irradiating step, or during microwave irradiation of the one or more mixed metal oxide precursor aerosols.

The process of the present invention may comprise the further step of further modifying the one or more metal oxide catalysts using one or more chemical treatments, one or more physical treatments and one or more combinations of chemical and physical treatments.

The present invention also provides a process for partial oxidation of an alkane, alkene, or a mixture of an alkane and an alkene, in the presence of a catalyst produced by the aforesaid process, wherein the process produces one or more partial oxidation products selected from the group consisting of an unsaturated carboxylic acid and an unsaturated nitrile.

The present invention provides a process for preparing mixed metal oxide catalysts by generating an aerosol from one or more solutions comprising mixed metal oxide precursors and irradiating the aerosol with microwave energy having one or more microwave frequencies.

More particularly, the precursor solutions may be sprayed through one or more ultrasonic devices (or any other comparable mechanical device that allows one to control the solution droplet size, including, but not limited to, ultrasonic spraying and ultrasonic expansion) into a microwave chamber where irradiation occurs. Alternatively, one or more precursor solutions may be sprayed through one or more ultrasonic devices (or any other comparable mechanical device that allows one to control the solution droplet size) and into the microwave chamber which already contains an additional precursor solution. In the latter case, precipitation/gelation will begin to take place upon addition of the sprayed precursor solution(s).

The frequency, dimensions, and power level (constant, oscillating, ramping, instantaneously maximizing, etc.) of the microwave device may be varied to optimize the catalyst's physical characteristics, composition, crystallinity, and the like. Both inorganic and organic templates may be used to control and direct the morphology of the catalyst precursor and final catalyst.

The aerosol may be fed into the microwave chamber, under turbulent flow conditions, where it may be irradiated, with microwave energy, at atmospheric temperature and/or pressure or, alternatively, at elevated temperature and/or pressure. Without intending to be limited by theory, it is believed that, by controlling the size of the aerosol droplets under turbulent flow, the resulting mixed metal oxide catalyst will have well-defined and uniform particle sizes, improved particle morphology and preferentially improved catalytic phases. IN particular, it is believed that the use of an ultrasonic spray or similar device allows one to control the droplet size(s) of the precursor solution(s). This, in turn, allows one to control the particle size, morphology, and potential crystallite plane exposure/surface area/porosity of the resulting mixed metal oxide precursor solid. The mixed metal oxide catalysts produced by this method are useful for converting alkanes, alkenes and mixtures thereof to their corresponding oxygenates, including unsaturated carboxylic acids and unsaturated nitriles. Furthermore, the resulting mixed metal oxide catalysts may be further improved by subjecting them to further chemical, physical and combinations of chemical and physical treatments (referred to as "post treatment" of prepared catalysts). Such methods and post treatments have resulted in unexpected improvements in catalyst efficiency and selectivity, as well as unexpected changes in catalyst properties, including catalyst structure, density and surface area.

Inventors have further discovered, for example, that the mixed metal oxide catalysts prepared by the method of the present invention unexpectedly provide improved selectivities and yields of oxygenates including unsaturated carboxylic acids from their corresponding alkanes at constant alkane conversion.

According to one embodiment, the mixed metal oxide prepared by the method of the present invention has the empirical formula:

MoVₐNb_{b}X_{c}Z_{d}Oₙ

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements. The performance of an Mo-V-Nb-Te-based mixed metal oxide catalyst may, for example, be optimized by increasing its surface area, porosity, maximizing the [001] plane exposure, and minimizing the aspect ratio, each of which may be achieved by the process of the present invention wherein a precursor-containing aerosol is irradiated with microwave energy.

As used herein, mixed metal oxide catalyst refers to a catalyst comprising more than one metal oxide. The term "catalytic system" refers to two or more catalysts. For example, platinum metal and indium oxide impregnated on an alumina support defines both a catalytic system and a mixed metal oxide catalyst. Yet another example of both is palladium metal, vanadium oxide and magnesium oxide impregnated on silica.

This new synthesis approach is useful for the optimization of known mixed metal oxide compositions (MMOs), such as, without limitation, Mo-V-Te-Nb-based mixed metal oxide catalysts, Mo-V-Sb-Nb-based mixed metal oxide catalysts, as well as three component MMOs (e.g., Mo-V-Te-Ox and Mo-V-Nb-Ox). It is also useful for the synthesis of new MMOs that may serve as selective oxidation catalysts (e.g., the conversion of propane to acrylic acid).

The process of the present invention may be conducted such that the catalyst precursor(s) are dried and converted to the active, calcined catalyst by controlling a number of experimental parameters including the energy type, frequency, power level, energy pulse type, reactor dimensions, residence time, gas environment, flow rate, temperature, gas feed additives, and the like. Conducting the drying and calcination *in-situ* has a number of advantages including improving the processing economics, providing catalyst particles of a more preferred shape, surface morphology, particle size range, crystallite phase(s), surface area, porosity, surface composition, and the like.

According to one embodiment of the invention, the catalysts prepared in accordance with the process of the present invention are one or more mixed metal oxide catalysts having a catalyst having the empirical formula

MoVₐNb_{b}X_{c}Z_{d}Oₙ

wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements. Preparation of the mixed metal oxide (MMO) catalysts is described in U. S. Patent Nos. 6,383,978; 6,641,996; 6,518,216; 6,403,525; 6,407,031; 6,407,280; and 6,589,907; U. S. Publication Application No. 20030004379; U. S. Provisional Application Ser. Nos. 60/235,977; 60/235,979; 60/235,981; 60/235,984; 60/235,983; 60/236,000; 60/236,073; 60/236,129; 60/236,143; 60/236,605; 60/236,250; 60/236,260; 60/236,262; 60/236,263; 60/283,245; and 60/286,218; and EP Patent Nos. EP 1 080 784; EP 1 192 982; EP 1 192 983; EP 1 192 984; EP 1 192 986; EP 1 192 987; EP 1 192 988; EP 1 192 982; EP 1 249 274; and EP 1 270 068.

It is noted that promoted mixed metal oxides having the empirical formulae MoⱼVₘTeₙNb_{y}Z_{z}Oₒ or WⱼVₘTeₙNb_{y}Z_{z}Oₒ, wherein Z, j, m, n, y, z and o are as previously defined, are particularly suitable for use in connection with the present invention. Additional suitable embodiments are either of the aforesaid empirical formulae, wherein Z is Pd. Suitable solvents for the precursor solution include water; alcohols including , but not limited to, methanol, ethanol, propanol, and diols, etc.; as well as other polar solvents known in the art. Generally, water is preferred. The water is any water suitable for use in chemical syntheses including, without limitation, distilled water and de-ionized water. The amount of water present is preferably an amount sufficient to keep the elements substantially in solution long enough to avoid or minimize compositional and/or phase segregation during the preparation steps. Accordingly, the amount of water will vary according to the amounts and solubilities of the materials combined. Preferably, though lower concentrations of water are possible for forming a slurry, as stated above, the amount of water is sufficient to ensure an aqueous solution is formed, at the time of mixing.

According to a separate embodiment of the invention, suitable prepared mixed metal oxide catalysts prepared in accordance with the process of the present invention may be one or more promoted mixed metal oxide catalysts having the empirical formula

JⱼMₘNₙY_{y}Z_{z}Oₒ

wherein J is at least one element selected from the group consisting of Mo and W, M is at least one element selected from the group consisting of V and Ce, N is at least one element selected from the group consisting of Te, Sb and Se, Y is at least one element selected from the group consisting of Nb, Ta, Ti, Al, Zr, Cr, Mn, Fe, Ru, Co, Rh, Ni, Pt, Sb, Bi, B, In, As, Ge, Sn, Li, Na, K, Rb, Cs, Fr, Be, Mg, Ca, Sr, Ba, Ra, Hf, Pb, P, Pm, Eu, Gd, Dy, Ho, Er, Tm, Yb and Lu, and Z is selected from the group consisting of Ni, Pd, Cu, Ag and Au; and wherein, when j = 1, m = 0.01 to 1.0, n = 0.01 to 1.0, y = 0.01 to 1.0, z = 0.001 to 0.1 and o is dependent on the oxidation state of the other elements. Preparation of the mixed metal catalysts is described in U. S. Patent Nos. 6,383,978; 6,641,996; 6,518,216; 6,403,525; 6,407,031; 6,407,280; and 6,589,907; U. S. Provisional Application Ser. Nos. 60/235,977; 60/235,979; 60/235,981; 60/235,984; 60/235,983; 60/236,000; 60/236,073; 60/236,129; 60/236,143; 60/236,605; 60/236,250; 60/236,260; 60/236,262; 60/236,263; 60/283,245; and 60/286,218; and EP Patent Nos. EP 1 080 784; EP 1 192 982; EP 1 192 983; EP 1 192 984; EP 1 192 986; EP 1 192 987; EP 1 192 988; EP 1 192 982; and EP 1 249 274.

Any conventional equipment and methods used for generating an aerosol is usefully employed in accordance with the invention. According to one embodiment, the aersol of mixed metal catalyst precursors is generated under conditions of turbulent flow. According to a separate embodiment, the aerosol is generated by an ultrasonic nozzle, which allows for control of the aerosol droplet size.

One of the problems encountered during the development of the spray drying method for the production of mixed-oxide catalysts of Mo, V, Nb, Sb and Te was the plugging of the spray drying nozzle. While this issue has been minimized by the delayed mixing and reaction of the starting solutions, by the cooling of the spray nozzle and by the use of larger diameter spray nozzle tips, plugging continues to be an intermittent problem during our spray drying operations. Furthermore, the use of large diameter spray nozzle tips also results in precursor gel droplets with large diameters which in turn require longer times to dry.

In order to further minimize the plugging problems associated with the production of precursors of mixed-oxide catalysts of Mo, V, Nb, Sb and Te as well as to extend the range of the sizes of precursor gel droplets produced in the spray dryer into the micrometer range we are proposing the use of ultrasonic atomizing nozzles.

Ultrasonic atomizing nozzles have many desirable features for spray drying. They include tight droplet size distribution, a low velocity spray, ability to control droplet size by changing nozzle frequency, a non-clogging nozzle, short set-up times, ability to run very small batch sizes, multiple liquid feeding capability, and a high degree of flexibility.

Some ultrasonic nozzles operate by converting a high frequency electrical signal, fed into two electrodes sandwiched between two piezo electric transducers, resulting in mechanical expansion and contraction of the transducers. This causes vibrations to be sent down the nozzle's titanium horn, ultrasonically vibrating at the nozzle's atomizing tip. Liquid emerging onto the atomizing surface is broken into a spray by the ultrasonic energy concentrated there. This ultrasonic nozzle design provides an easily controllable atomized spray that cannot clog because of the large flux feed orifice and the self-cleaning ultrasonic vibration. Droplet diameter may be controlled according to the frequency used in the nozzle, for example, 25 kHz frequency provides an approximately 70 µ droplet diameter, 48kHz frequency provides an approximately 38 µ droplet diameter, 60kHz frequency provides an approximately 31 µ droplet diameter, and 120kHz frequency provides an approximately 18 µ droplet diameter. Dual liquid feed allows for even greater flexibility in the process, as two liquids can be mixed right at the nozzle tip during atomization. Such nozzles are commercially available from a number of sources, including Sono-Tek Corporation, of Milton, New York, U.S.A.

It is also possible for ultrasonic nozzles to use compressed air or gas to energize the nozzle; therefore, there is no piezoelectric effect or electricity needed. A sonic field is created at the throat of the nozzle as the compressed gas accelerates and reaches the velocity of sound. High frequency waves created by the resonator cavity produce a chopping effect that breaks the liquid stream into a fine, evenly dispersed cloud of extremely small droplets. These nozzles can produce droplet sizes significantly below those of conventional air atomizers - mean droplet diameters of as little as 8 microns are obtained when spraying water in certain nozzles. This makes these nozzles ideal for use in spray drying and other applications that require extremely small droplets. Such nozzles are commercially available from a number of sources, including Sono-Tek Corporation, of Milton, New York, U.S.A.

The application of ultrasonic nozzles in the spray drying production of 2-, 3⁻, 4-, and 5-component combinations of precursors of mixed-oxide catalysts containing Mo, V, Nb, Sb and Te, with and without the addition of supports such as silica, is expected to result in precursor particle sizes in the nano to the micrometer range, depending on a number of factors, such as, but not limited to, the concentration of the starting materials solutions used in the process. Furthermore, the primary spray dried particles obtained by such methods are in general comprised of an agglomeration of smaller particles and/or crystallites. Therefore, the use of ultrasonic nozzles with standard spray drying conditions is expected to allows us to produce catalyst crystallites with sizes and shapes previously unavailable by standard spray drying.

The mechanical simplicity of the aforesaid ultrasonic nozzles, the well-established simplicity and low operational cost of standard spray drying, and the ability to use air, water and standard starting materials in the synthesis of nano-and micron-size particles, present an economic and operational advantage over other methods that have been proposed in the past to achieve the same goal. For example, spray drying under supercritical conditions requires compression, high pressures, use and recycle of CO₂, high ratios of an anti-solvent, such as alcohols, when aqueous solutions are used, and the use of expensive and/or exotic starting materials, such as alkoxides, when non-aqueous solutions are preferred. Flame processes, such as nGimat's NanoSpray^{SM} process, also require more complex operational and experimental configuration, the use of fuels and high temperatures, limited control of the reduction potential of the environment where the particles are formed, as well as the use of starting materials and solutions that can be combusted.

Finally, nano- and micro-scale precursor and catalyst particles produced by the use of ultrasonic nozzles in conjunction with standard spray drying are suitable for further processing such as calcination or for direct use in fluid bed reactors. Ultrasonic nozzles are also ideal for use in other applications that require extremely small droplets such as, but not limited to, evaporative cooling and spray coating.

The frequency, dimensions, and power level (constant, oscillating, ramping, instantaneously maximizing, etc.) of the microwave device may be varied to optimize the catalyst's physical characteristics, composition, crystallinity, and the like. Both inorganic and organic templates may be used to control and direct the morphology of the catalyst precursor and final catalyst.

A mixed metal oxide catalyst (promoted or not), thus obtained, exhibits excellent catalytic activities by itself. However, the mixed metal oxide catalyst may be converted to a modified catalyst having higher activities by one or more chemical, physical and combinations of chemical and physical treatments.

As used herein, the term "modified catalysts" is equivalent to the term "post-treated catalysts" and both refer to any chemical, physical, or combination of chemical and physical, modification or modifications of one or more mixed metal oxide catalysts as compared to corresponding catalysts of similar composition which have not undergone such modification or modifications (also referred to as "known catalysts"). Modifications to mixed metal oxide catalysts include, but are not limited to, any differences in the modified catalysts as compared to corresponding known catalysts. Suitable modifications to catalysts include, for example, without limitation, structural changes, spectral changes (including position and intensity of characteristic X-ray diffraction lines, peaks and patterns), spectroscopic changes, chemical changes, physical changes, compositional changes, changes in physical properties, changes in catalytic properties, changes in performance characteristics in conversions of organic molecules, changes in yields of organic products from corresponding reactants, changes in catalyst activity, changes in catalyst selectivity and combinations thereof. This includes one or more chemical modifying agents (e.g. a reducing agent such as an amine), one or more physical processes (e.g. mechanical grinding at cryogenic temperatures also referred to as "cryo-grinding") and combinations of one or more chemical modifying agents and one or more physical processes. The term "cryo" in front of any treatment term refers to any treatment that occurs with cooling, under freezing temperatures, at cryogenic temperatures and any use of cryogenic fluids. Suitable cryogenic fluids include, but are not limited to for example, any conventional cryogens and other coolants such as chilled water, ice, compressible organic solvents such as freons, liquid carbon dioxide, liquid nitrogen, liquid helium and combinations thereof.

Suitable chemical and physical modification of the catalysts prepared in accordance with the process of the present invention may result in unexpected improvements in modified catalyst efficiency and selectivity in alkane, alkene or alkane and alkene oxidations as compared to corresponding known catalysts and improved yields of oxygenated products. The known catalysts may be obtained from commercial sources, conventional preparation methods, or by any of the methods of the present invention.

The term "modified catalysts" does not refer to or include regenerated, reconditioned and/or recycled catalysts. The term conditioning refers to conventional heating of prepared metal oxide catalysts with gases including hydrogen, nitrogen, oxygen and selected combinations thereof.

As used herein, the term "cumulatively converting" refers producing a desired product stream from one or more specific reactants using one or more modified catalysts and modified catalyst systems of the invention under specific reaction conditions. As an illustrative embodiment, cumulatively converting an alkane to its corresponding unsaturated carboxylic acid means that the modified catalyst(s) utilized will produce a product stream comprising the unsaturated carboxylic acid corresponding to the added alkane when acting on a feed stream(s) comprising the alkane and molecular oxygen under the designated reaction conditions. According to a separate embodiment, the present invention also provides a process for optimizing recycle conversion of specific alkanes, alkenes, alkanes and alkenes and their corresponding oxygenate products.

Optionally, modified metal oxide catalysts are obtained by treating chemical, physical and combinations of chemical and physical treatments of suitable prepared metal oxide catalyst. Optionally, the modified catalysts are further modified by conventional processing techniques well known to persons having skill in this art.

Chemical treatments, resulting in modified catalysts, include one or more chemical modifying agents. Physical treatments, resulting in modified catalysts, include one or more physical processes. According to a separate embodiment, modified catalysts include one or more further chemical and/or physical treatments of already modified catalysts.

Once obtained, the resulting modified catalyst precursor is used as modified or is further modified by conventional processes well known in the art, including further milling and calcining.

According to one embodiment, calcination may be conducted in an oxygen-containing atmosphere or in the substantial absence of oxygen, e.g., in an inert atmosphere or in vacuo. The inert atmosphere may be any material which is substantially inert, i.e., does not react or interact with, the catalyst precursor. Suitable examples include, without limitation, nitrogen, argon, xenon, helium or mixtures thereof. Preferably, the inert atmosphere is argon or nitrogen. The inert atmosphere may flow over the surface of the catalyst or may not flow thereover (a static environment). When the inert atmosphere does flow over the surface of the catalyst precursor, the flow rate can vary over a wide range, e.g., at a space velocity of from 1 to 500 hr¹.

Calcination of MMO catalysts such as those described hereinabove is usually performed at a temperature of from 350°C to 850°C, preferably from 400°C to 700°C, more preferably from 500°C to 640°C. The calcination is performed for an amount of time suitable to form the aforementioned catalyst. Typically, the calcination is performed for from 0.5 to 30 hours, preferably from 1 to 25 hours, more preferably for from 1 to 15 hours, to obtain the desired promoted mixed metal oxide.

According to one embodiment, the catalyst is calcined in two stages. In the first stage, the catalyst precursor is calcined in an oxidizing environment (e.g. air) at a temperature of from 200°C to 400°C, preferably from 275°C to 325°C for from 15 minutes to 8 hours, preferably for from 1 to 3 hours. In the second stage, the material from the first stage is calcined in a non-oxidizing environment (e.g., an inert atmosphere) at a temperature of from 500°C to 700°C, preferably for from 550°C to 650°C, for 15 minutes to 8 hours, preferably for from 1 to 3 hours. Optionally, a reducing gas, such as, for example, ammonia or hydrogen, may be added during the second stage calcination.

The MMO catalyst (promoted or not) obtained by the above-mentioned method may be used as a final catalyst, but it may further be subjected to one or more additional chemical, physical and combinations of chemical and physical treatments. According to one embodiment, the MMO catalysts produced in accordance with the process of the present invention are further modified using heat treatment. As an exemplary embodiment, heat treatment usually is performed at a temperature of from 200° to 700°C for from 0.1 to 10 hours.

The resulting modified mixed metal oxide (promoted or not) may be used by itself as a solid catalyst. The modified catalysts are also combined with one or more suitable carriers, such as, without limitation, silica, alumina, titania, aluminosilicate, diatomaceous earth or zirconia, according to art-disclosed techniques. Further, it may be processed to a suitable shape or particle size using art disclosed techniques, depending upon the scale or system of the reactor.

Alternatively, the metal components of the modified catalysts are supported on materials such as alumina, silica, silica-alumina, zirconia, titania, etc. by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal-containing support is then dried and calcined as detailed above.

According to a separate embodiment, modified catalysts are also prepared using one or more promoters. The starting materials for the above promoted mixed metal oxide are not limited to those described above. A wide range of materials including, for example, oxides, nitrates, halides or oxyhalides, alkoxides, acetylacetonates, and organometallic compounds may be used. For example, ammonium heptamolybdate may be utilized for the source of molybdenum in the catalyst. However, compounds such as MoO₃, MoO₂, MoCl₅, MoOCl₄, Mo(OC₂H₅)₅, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as V₂O₅, V₂O₃, VOCl₃, VCl₄, VO(OC₂H₅)₃, vanadium acetylacetonate and vanadyl acetylacetonate may also be utilized instead of ammonium metavanadate. The tellurium source may include telluric acid, TeCl₄, Te(OC₂H₅)₅, Te(OCH(CH₃)₂)₄ and TeO₂. The niobium source may include ammonium niobium oxalate, Nb₂O₅, NbCl₅, niobic acid or Nb(OC₂H₅)₅ as well as the more conventional niobium oxalate.

In addition, with reference to the promoter elements for the promoted catalyst, the nickel source may include nickel(II) acetate tetrahydrate, Ni(NO₃)₂, nickel(II) oxalate, NiO, Ni(OH)₂, NiCl₂, NiBr₂, nickel(II) acetylacetonate, nickel(II) sulfate, NiS or nickel metal. The palladium source may include Pd(NO₃)₂, palladium(II) acetate, palladium oxalate, PdO, Pd(OH)₂, PdCl₂, palladium acetylacetonate or palladium metal. The copper source may be copper acetate, copper acetate monohydrate, copper acetate hydrate, copper acetylacetonate, copper bromide, copper carbonate, copper chloride, copper chloride dihydrate, copper fluoride, copper formate hydrate, copper gluconate, copper hydroxide, copper iodide, copper methoxide, copper nitrate, copper nitrate hydrate, copper oxide, copper tartrate hydrate or a solution of copper in an aqueous inorganic acid, e.g., nitric acid. The silver source may be silver acetate, silver acetylacetonate, silver benzoate, silver bromide, silver carbonate, silver chloride, silver citrate hydrate, silver fluoride, silver iodide, silver lactate, silver nitrate, silver nitrite, silver oxide, silver phosphate or a solution of silver in an aqueous inorganic acid, e.g., nitric acid. The gold source may be ammonium tetrachloroaurate, gold bromide, gold chloride, gold cyanide, gold hydroxide, gold iodide, gold oxide, gold trichloride acid and gold sulfide.

MMO catalysts prepared in accordance with the process of the present invention have different chemical, physical and performance characteristics in catalytic reactions of carbon based molecules as compared to other catalysts. According to one embodiment, the modified catalyst exhibits changes in X-ray lines, peak positions and intensity of such lines and peaks as compared with corresponding X-ray diffraction data for corresponding other catalysts. Such difference indicate structural differences between known catalysts and catalysts of the present invention are evident from the catalytic activity and selectivity.

MMO catalysts prepared in accordance with the process of the present invention exhibit improved catalyst performance characteristics selected from the group consisting of optimized catalyst properties, yields of oxygenates including unsaturated carboxylic acids, from their corresponding alkanes, alkenes or combinations of corresponding alkanes and alkenes at constant alkane/alkene conversion, selectivity of oxygenate products, including unsaturated carboxylic acids, from their corresponding alkanes, alkenes or combinations of corresponding alkanes and alkenes, optimized feed conversion, cumulative yield of the desired oxidation product, optimized reactant/product recycle conversion, optimized product conversion via recycle and combinations thereof, as compared to the known catalyst.

MMO catalysts prepared in accordance with the process of the present catalysts of the invention have improved performance characteristics as compared to known catalysts in catalytic processes comprising any carbon containing molecule. According to one embodiment of the invention, the modified catalysts have improved performance characteristics as compared to known catalysts in processes for preparing dehydrogenated products and oxygenated products from alkanes and oxygen, alkenes and oxygen and combination of alkanes, alkenes and oxygen. The reactions are typically carried out in conventional reactors with the alkanes catalytically converted at conventional residence times (> 100 milliseconds) in conventional reactors. According to a separate embodiment the reactions are carried out at short contact times (≤ 100 milliseconds) in a short contact time reactor. Suitable alkanes include alkanes having straight or branched chains. Examples of suitable alkanes are C₂-C₂₅ alkanes, including C₂-C₈ alkanes such as propane, butane, isobutane, pentane, isopentane, hexane and heptane. Particularly preferred alkanes are propane and isobutane.

MMO catalysts prepared in accordance with the process of the present catalysts of the invention convert alkanes, alkenes or alkanes and alkenes to their corresponding alkenes and oxygenates including saturated carboxylic acids, unsaturated carboxylic acids, esters thereof, and higher analogue unsaturated carboxylic acids and esters thereof. The catalyst and catalytic systems produced by the process of the present invention are designed to provide specific alkenes, oxygenates and combinations thereof. Alkanes are catalytically converted to one or more products in a single pass, including corresponding alkenes. Any unreacted alkane, alkene or intermediate is recycled to catalytically convert it to its corresponding oxygenate. According to one embodiment, alkenes produced from dehydrogenation of corresponding alkanes using catalyst systems of the invention are deliberately produced as in-process chemical intermediates and not isolated before selective partial oxidation to oxygenated products. For example, when catalytically converting an alkane to its corresponding ethylenically unsaturated carboxylic acid, any unreacted alkene produced is recovered or recycled to catalytically convert it to its corresponding ethylenically unsaturated carboxylic acid product stream.

Catalysts and catalyst systems produced by the process of the present invention also convert alkanes to their corresponding ethylenically unsaturated carboxylic acids and higher analogues having straight or branched chains. Examples include C₂-C₈ ethylenically unsaturated carboxylic acids such as acrylic acid, methacrylic acid, butenoic acid, pentenoic acid, hexenoic acid, maleic acid, and crotonic acid. Higher analogue ethylenically unsaturated carboxylic acids are prepared from corresponding alkanes and aldehydes. For example, methacrylic acid is prepared from propane and formaldehyde. According to a separate embodiment, the corresponding acid anhydrides are also produced when preparing ethylenically unsaturated carboxylic acids from their respective alkanes. The modified catalysts of the invention are usefully employed to convert propane to arcylic acid and its higher unsaturated carboxylic acid methacrylic acid and to convert isobutane to methacrylic acid.

Catalysts and catalyst systems produced by the process of the present invention are also advantageously utilized converting alkanes to their corresponding esters of unsaturated carboxylic acids and higher analogues. Specifically, these esters include, but are not limited to, butyl acrylate from butyl alcohol and propane, β-hydroxyethyl acrylate from ethylene glycol and propane, methyl methacrylate from methanol and isobutane, butyl methacrylate from butyl alcohol and isobutane, β-hydroxyethyl methacrylate from ethylene glycol and isobutane, and methyl methacrylate from propane, formaldehyde and methanol.

In addition to these esters, other esters are formed through this invention by varying the type of alcohol introduced into the reactor and/or the alkane, alkene or alkane and alkene introduced into the reactor.

Suitable alcohols include monohydric alcohols, dihydric alcohols and polyhydric alcohols. Of the monohydric alcohols reference may be made, without limitation, to C₁-C₂₀ alcohols, preferably C₁-C₆ alcohols, most preferably C₁-C₄ alcohols. The monohydric alcohols may be aromatic, aliphatic or alicyclic; straight or branched chain; saturated or unsaturated; and primary, secondary or tertiary. Particularly preferred monohydric alcohols include methyl alcohol, ethyl alcohol, propyl alcohol, isopropyl alcohol, butyl alcohol, isobutyl alcohol and tertiary butyl alcohol. Of the dihydric alcohols reference may be made, without limitation, to C₂-C₆ diols, preferably C₂-C₄ diols. The dihydric alcohols may be aliphatic or alicyclic; straight or branched chain; and primary, secondary or tertiary. Particularly preferred dihydric alcohols include ethylene glycol (1,2-ethanediol), propylene glycol (1,2-propanediol), trimethylene glycol (1,3-propanediol), 1,2-butanediol and 2,3-butanediol. Of the polyhydric alcohols reference will only be made to glycerol (1,2,3-propanetriol).

The unsaturated carboxylic acid corresponding to the added alkane is the α,β-unsaturated carboxylic acid having the same number of carbon atoms as the starting alkane and the same carbon chain structure as the starting alkane, e.g., acrylic acid is the unsaturated carboxylic acid corresponding to propane and methacrylic acid is the unsaturated carboxylic acid corresponding to isobutane.

The mixed metal oxide thus obtained is typically used by itself as a solid catalyst, but may be formed into a catalyst together with a suitable carrier such as silica, alumina, titania, aluminosilicate, diatomaceous earth or zirconia. Further, it may be molded into a suitable shape and particle size depending upon the scale or system of the reactor.

Alternatively, the metal components of the modified catalysts may be supported on materials such as alumina, silica, silica-alumina, zirconia, titania, etc. by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal containing support is then dried and calcined as detailed above.

When using a catalyst system including two or more modified catalysts, the catalyst may be in the form of a physical blend of the several catalysts. Preferably, the concentration of the catalysts may be varied so that the first catalyst component will have a tendency to be concentrated at the reactor inlet while subsequent catalysts will have a tendency to be concentrated in sequential zones extending to the reactor outlet. Most preferably, the catalysts will form a layered bed (also referred to a mixed bed catalyst), with the first catalyst component forming the layer closest to the reactor inlet and the subsequent catalysts forming sequential layers to the reactor outlet. The layers abut one another or may be separated from one another by a layer of inert material or a void space.

The invention provides a process for producing an unsaturated carboxylic acid, which comprises subjecting an alkane, alkene or a mixture of an alkane and an alkene ("alkane/alkene"), to a vapor phase catalytic oxidation reaction in the presence of a catalyst containing the above promoted mixed metal oxide, to produce an unsaturated carboxylic acid.

In the production of such an unsaturated carboxylic acid, it is preferred to employ a starting material gas that contains steam. In such a case, as a starting material gas to be supplied to the reaction system, a gas mixture comprising a steam-containing alkane, or a steam-containing mixture of alkane and alkene, and an oxygen-containing gas, is usually used. However, the steam-containing alkane, or the steam-containing mixture of alkane and alkene, and the oxygen-containing gas may be alternately supplied to the reaction system. The steam to be employed may be present in the form of steam gas in the reaction system, and the manner of its introduction is not particularly limited.

Further, as a diluting gas, an inert gas such as nitrogen, argon or helium may be supplied. The molar ratio (alkane or mixture of alkane and alkene) : (oxygen) : (diluting gas) : (H₂O) in the starting material gas is preferably (1) : (0.1 to 10): (0 to 20) : (0.2 to 70), more preferably (1) : (1 to 5.0) : (0 to 10) : (5 to 40).

When steam is supplied together with the alkane, or the mixture of alkane and alkene, as starting material gas, the selectivity for an unsaturated carboxylic acid is distinctly improved, and the unsaturated carboxylic acid can be obtained from the alkane, or mixture of alkane and alkene, in good yield simply by contacting in one stage. However, the conventional technique utilizes a diluting gas such as nitrogen, argon or helium for the purpose of diluting the starting material. As such a diluting gas, to adjust the space velocity, the oxygen partial pressure and the steam partial pressure, an inert gas such as nitrogen, argon or helium may be used together with the steam.

As the starting material alkane it is preferred to employ a C₂₋₈ alkane, particularly propane, isobutane or n-butane; more preferably, propane or isobutane; most preferably, propane. According to the present invention, from such an alkane, an unsaturated carboxylic acid such as an α,β-unsaturated carboxylic acid can be obtained in good yield. For example, when propane or isobutane is used as the starting material alkane, acrylic acid or methacrylic acid will be obtained, respectively, in good yield.

This aspect present invention is described in still further detail with respect to a case where propane is used as the starting material alkane and air is used as the oxygen source. The reaction system may be preferably a fixed bed system. The proportion of air to be supplied to the reaction system is important for the selectivity for the resulting acrylic acid, and it is usually at most 25 moles, preferably from 0.2 to 18 moles per mole of propane, whereby high selectivity for acrylic acid can be obtained. This reaction can be conducted usually under atmospheric pressure, but may be conducted under a slightly elevated pressure or slightly reduced pressure. With respect to other alkanes such as isobutane, or to mixtures of alkanes and alkenes such as propane and propene, the composition of the feed gas may be selected in accordance with the conditions for propane.

Typical reaction conditions for the oxidation of propane or isobutane to acrylic acid or methacrylic acid may be utilized in the practice of the present invention. The process may be practiced in a single pass mode (only fresh feed is fed to the reactor) or in a recycle mode (at least a portion of the reactor effluent is returned to the reactor). General conditions for the process of the present invention are as follows: the reaction temperature can vary from 200°C to 700°C, but is usually in the range of from 200°C to 550°C, more preferably 250°C to 480°C, most preferably 300°C to 400°C; the gas space velocity, SV, in the vapor phase reaction is usually within a range of from 100 to 10,000 hr⁻¹, preferably 300 to 6,000 hr⁻¹, more preferably 300 to 2,000 hr⁻¹; the average contact time with the catalyst can be from 0.01 to 10 seconds or more, but is usually in the range of from 0.1 to 10 seconds, preferably from 0.2 to 6 seconds; the pressure in the reaction zone usually ranges from 0 to 75 psig, but is preferably no more than 50 psig. In a single pass mode process, it is preferred that the oxygen be supplied from an oxygen-containing gas such as air. The single pass mode process may also be practiced with oxygen addition. In the practice of the recycle mode process, oxygen gas by itself is the preferred source so as to avoid the build up of inert gases in the reaction zone. The feed of hydrocarbon in the catalytic process is dependent on the mode of operation (e.g. single pass, batch, recycle, etc.) and ranges from 2 wt. % to 50 wt. %. According to a separate embodiment, the catalytic process is a batch process. According to a separate process, the catalytic process is run continuously. The catalytic process all conventional beds including, but not limited to static fluid beds, fluidized beds, moving beds, transport beds, moving beds such as rising and ebulating beds. Any catalytic process is carried out under steady state conditions or non steady state conditions.

The following illustrative examples are provided to further demonstrate the utility of the present invention and are not in any way construed to be limiting. Moreover, the examples provided are representative examples that broadly enable the claimed scope of the invention. In the following Examples, "propane conversion" is synonymous with "feed conversion" and was calculated in accordance with the formulas provided earlier hereinabove. Furthermore, "AA yield" means acrylic acid yield and is synonymous with "product yield" and was calculated in accordance with the formulas provided earlier hereinabove.

Unless otherwise specified, all percentages recited in the following Examples are by volume, based on the total volume of the feed or product gas stream.

### EXAMPLES

### Comparative Examples 1A-1G (synthesis with microwave irradiation)

Ammonium heptamolybdate, ammonium vanadate and telluric acid were used to prepare a Solution A, in the following amounts: 1 M Mo, 0.3 M V and 0.23 M Te. Niobium ammonium oxalate, oxalic acid dehydrate, palladium (II) nitrate hydrate, and nitric acid were used to prepare a Solution B, in the following amounts: 0.17 M Nb, 0.155 M oxalic acid, 0.24 M HNO₃ and 0.01 M Pd. Alternative sources for V and Te include vanadyl sulfate hydrate and tellurium oxide.

A catalyst of nominal composition Mo_{1.0}V_{0.3}Te_{0.23}Nb_{0.17}Pd_{0.01}Oₓ was prepared by mixing the aforesaid solutions A and B in bulk inside a reactor. The bulk mixture was then irradiated with microwaves. The resulting gel was dried at room temperature. The dried sample was calcined under air at 25°C to 275°C at 10°C/min and held at 275°C for 1 hour, and then under argon at 275°C to 600°C at 2°C/min and held at 600°C for 2 hours. The catalyst, thus obtained, was pressed in a mold and then broken and sieved to 10 - 20 mesh granules.

5.8 g of the aforesaid catalyst were packed into a stainless steel straight down flow (SDF) tube reactor (inside diameter of 1.1 cm) for the gas phase oxidation of propane. The SDF reactor was placed in a furnace and fed with a mixture of propane, air and steam having a feed composition of 7% propane, 14.7% oxygen (in air) and 23% steam. The effluent of the reactor was condensed to a separate a liquid phase and a gas phase. The gas phase was analyzed by gas chromatography to determine the propane conversion. The liquid phase was also analyzed by gas chromatography for the yield of acrylic acid. The aforesaid evaluation of the catalyst in the SDF reactor system was repeated four times. The results along with residence time and reactor temperature are shown in Table 1.

**Table 1**

| Comparative Examples | Residence Time (sec) | Temp. (°C) | Propane Conv. (%) | Acrylic Acid Selectivity (%) | Acrylic Acid Yield (%) |
|---|---|---|---|---|---|
| Comp. Ex. 1A | 3 | 309 | 8.7 | 59 | 5.1 |
| Comp. Ex. 1B | 3 | 330 | 14 | 61 | 8.6 |
| Comp. Ex. 1C | 3 | 351 | 21 | 67 | 14 |
| Comp. Ex. 1D | 3 | 374 | 33 | 67 | 22 |
| Comp. Ex. 1E | 3 | 390 | 43 | 62 | 27 |
| Comp. Ex. 1F | 3 | 404 | 53 | 55 | 29 |
| Comp. Ex. 1G | 3 | 415 | 61 | 44 | 27 |

### Examples 2A-2F (synthesis by preparing an aerosol comprising precursor solutions and irradiating with microwave energy)

Ammonium heptamolybdate, ammonium vanadate and telluric acid were used to prepare a Solution A, in the following amounts: 1 M Mo, 0.3 M V and 0.23 M Te. Niobium ammonium oxalate, oxalic acid dehydrate, palladium (II) nitrate hydrate, and nitric acid were used to prepare a Solution B, in the following amounts: 0.17 M Nb, 0.155 M oxalic acid, 0.24 M HNO₃ and 0.01 M Pd. Alternative sources for V and Te include vanadyl sulfate hydrate and tellurium oxide.

A catalyst of nominal composition Mo_{1.0}V_{0.3}Te_{0.23}Nb_{0.17}Pd_{0.01}Oₓ was prepared in accordance with the process of the present invention as follows. Solutions A and B were mixed i*n situ* by passage through an ultrasound nozzle device, using two syringes, a "T" connector and a syringe pump. The resulting mixture was injected into a tubular reactor inside a microwave chamber using a nozzle spray, which resulted in formation of small droplets, which then formed a gel of the precursor mixture. The resulting gel was dried at room temperature. The dried sample was calcined under air at 25°C to 275°C at 10°C/min and held at 275°C for 1 hour, and then under argon at 275°C to 600°C at 2°C/min and held at 600°C for 2 hours.

5.1 g of the aforesaid catalyst were packed into a stainless steel straight down flow (SDF) tube reactor (inside diameter of 1.1 cm) for the gas phase oxidation of propane. The SDF reactor was placed in a furnace and fed with a mixture of propane, air and steam having a feed composition of 6.9% propane, 14.7% oxygen (in air) and 23% steam. The effluent of the reactor was condensed to a separate a liquid phase and a gas phase. The gas phase was analyzed by gas chromatography to determine the propane conversion. The liquid phase was also analyzed by gas chromatography for the yield of acrylic acid. The aforesaid evaluation of the catalyst in the SDF reactor system was repeated six times. The results along with residence time and reactor temperature are shown in Table 2.

**Table 2**

| Examples | Residence Time (sec) | Temp. (°C) | Propane Conv. (%) | Acrylic Acid Selectivity (%) | Acrylic Acid Yield (%) |
|---|---|---|---|---|---|
| Ex. 2A | 3 | 321 | 17 | 67 | 11 |
| Ex. 2B | 3 | 342 | 26 | 68 | 17 |
| Ex. 2C | 3 | 364 | 39 | 69 | 27 |
| Ex. 2D | 3 | 378 | 49 | 66 | 33 |
| Ex. 2E | 3 | 390 | 60 | 59 | 35 |
| Ex. 2F | 3 | 395 | 65 | 54 | 35 |

The foregoing examples demonstrate that the catalyst of Comparative Examples 1A-1G and Examples 2A-2F were both successful in the vapor phase oxidation of an alkane to partial oxidation products. However the catalyst produced by the process wherein the catalyst precursors are first mixed and atomized to an aerosol using an ultrasound nozzle, and then irradiated with microwave energy, i.e., Examples 2A-2F, performed better in that higher acrylic acid yields were achieved.

It is noted that X-ray diffraction (XRD) analysis of the catalyst of Comparative Examples 1A-1G and Examples 2A-2F suggested the presence of M1 phase in both sample. SEM analysis for both catalysts, respectively, showed a rod-like morphology. The rod-like morphology has been widely reported in the literature for these kind of materials prepared by the conventional hydrothermal methods. However, the particle dimensions of the microwave prepared materials prepared by both methods were significantly smaller. Both catalysts had particle sizes of about 0.2 µm to about 0.3 µm in diameter and approximately 1 µm in length. These dimensions were about 10 times smaller than conventional, hydrothermally prepared materials. Additionally, the use of an ultrasonication nozzle spray in the preparation of the catalyst of Examples 2A-2F, in accordance with the process of the present invention, appeared to have provided a more uniform environment for reaction, which resulted in more uniform morphologies than achieved for the catalyst of Comparative Examples 1A-1G (prepared by a bulk process and using microwave irradiation).

It will be understood that the embodiments of the present invention described hereinabove are merely exemplary and that a person skilled in the art may make variations and modifications without departing from the spirit and scope of the invention. All such variations and modifications are intended to be included within the scope of the present invention.

## Claims

1. A process for preparing one or more mixed metal oxide catalysts suitable for partial oxidation of alkanes, alkenes, and mixtures thereof, comprising the steps of:
a) generating one or more aerosols from one or more solutions comprising at least one metal oxide catalyst precursor; and
b) irradiating said one or more aqueous aerosols of said one or more mixed metal oxide precursors with microwave energy, having one or more microwave frequencies.

2. The process according to claim 1, wherein the one or more mixed metal oxide catalysts prepared comprises a compound having the empirical formula:
MoVₐNb_{b}X_{c}Z_{d}Oₙ
wherein X is at least one element selected from the group consisting of Te and Sb, Z is at least one element selected from the group consisting of W, Cr, Ta, Ti, Zr, Hf, Mn, Re, Fe, Ru, Co, Rh, Ni, Pd, Pt, Ag, Zn, B, Al, Ga, In, Ge, Sn, Pb, P, Bi, Y, rare earth elements and alkaline earth elements, 0.1 ≤ a ≤ 1.0, 0.01 ≤ b ≤ 1.0, 0.01 ≤ c ≤ 1.0, 0 ≤ d ≤ 1.0 and n is determined by the oxidation states of the other elements.

3. The process according to claim 1, further comprising the step of calcining said one or more mixed metal oxide catalysts after said irradiating step.

4. The process according to claim 1, wherein said one or more mixed metal oxide catalysts are calcined during microwave irradiation of the one or more mixed metal oxide precursor aerosols.

5. The process according to claim 1, wherein the one or more mixed metal oxide precursor aerosols are generated using an ultrasonic nozzle.

6. The process according to claim 1, further comprising the step of further modifying the one or more metal oxide catalysts using one or more chemical treatments, one or more physical treatments and one or more combinations of chemical and physical treatments.

7. A catalyst prepared by the process of Claim 1.

8. A process for partial oxidation of an alkane, alkene, or a mixture of an alkane and an alkene, in the presence of a catalyst produced by the process of Claim 1, wherein said process produces one or more partial oxidation products selected from the group consisting of an unsaturated carboxylic acid and an unsaturated nitrile.
